# EUROPEAN PATENT APPLICATION

(11) **EP 4 586 792 A1**
(43) Date of publication of application: **16.07.2025**
(21) Application number: 23924649.9
(22) Date of filing: 01.03.2023
(51) Int. Cl.: H10K 85/20, C07C 35/44

(54) **FULLERENE COMPOSITE MATERIAL AND PREPARATION METHOD THEREFOR, PEROVSKITE SOLAR CELL AND PREPARATION METHOD THEREFOR, AND ELECTRIC DEVICE**

(71) Applicant: Contemporary Amperex Technology (Hong Kong) Limited, Central (HK)
(72) Inventor: LIANG, Weifeng, Ningde, Fujian 352100 (CN); SU, Shuojian, Ningde, Fujian 352100 (CN); CHEN, Changsong, Ningde, Fujian 352100 (CN); TU, Bao, Ningde, Fujian 352100 (CN); LUAN, Bo, Ningde, Fujian 352100 (CN); GUO, Yongsheng, Ningde, Fujian 352100 (CN); CHEN, Guodong, Ningde, Fujian 352100 (CN); CHEN, Junchao, Ningde, Fujian 352100 (CN)
(74) Representative: Jacob, Reuben Ellis
(86) International application number: PCT/CN2023/079072
(87) International publication number: WO 2024/178679

(57) **Abstract**

This application relates to a fullerene composite material and a preparation method therefor, a perovskite solar cell and a preparation method therefor, and an electrical device. The fullerene composite material includes a host material and a doping material, where the host material includes at least one of a first fullerene and a first fullerene derivative, the doping material is selected from at least one of a second fullerene and a second fullerene derivative, and a molecular structure of the doping material is partially embedded in a lattice of the first fullerene and/or the first fullerene derivative.

## Description

### TECHNICAL FIELD

This application relates to the field of solar cell technologies, and in particular, to a fullerene composite material and a preparation method therefor, a perovskite solar cell and a preparation method therefor, and an electrical device.

### BACKGROUND

With the continuous development of solar cell research, perovskite solar cells have attracted wide attention due to their high photoelectric conversion efficiency and simple preparation processes.

In a structure of a perovskite solar cell, a fullerene and its derivatives are common electron acceptors. However, with the continuous deepening of perovskite solar cell research, a perovskite solar cell obtained by preparing an electron transport layer thereof using a pure fullerene and its derivative still has low photoelectric conversion efficiency.

### SUMMARY

According to embodiments of this application, this application provides a fullerene composite material, including a host material and a doping material, where the host material includes at least one of a first fullerene and a first fullerene derivative, the doping material is selected from at least one of a second fullerene and a second fullerene derivative, and a molecular structure of the doping material is partially embedded in a lattice of the first fullerene and/or the first fullerene derivative.

In this application, the host material including the first fullerene and/or the first fullerene derivative is doped with at least one of the second fullerene and the second fullerene derivative, which can improve conductivity of the fullerene composite material and improve photoelectric conversion efficiency of the perovskite solar cell.

In some implementations, a mass percentage of the doping material based on a total mass of the first fullerene and/or the first fullerene derivative and the doping material ranges from 0.0001% to 1%. If the mass percentage of the doping material is too small, a doping effect is poor, and if the mass percentage of the doping material is too large, an intrinsic structure of the host material may be excessively affected.

In some implementations, the first fullerene includes at least one of C50, C60 and C70, and the first fullerene derivative includes at least one of PC51BM, PC52BM, PC61BM, PC62BM, PC71BM, and PC72BM. The fullerenes and fullerene derivatives are widely available and are easy to obtain.

In some implementations, the second fullerene includes at least one of C50, C60 and C70, and the second fullerene derivative includes at least one of PC51BM, PC52BM, PC61BM, PC62BM, PC71BM, and PC72BM. The fullerenes and fullerene derivatives are widely available and are easy to obtain.

This application further provides a preparation method for a fullerene composite material, including the following step:
mixing a first raw material and a second raw material in a first solvent, and performing heat treatment on an obtained mixed system, where
the first raw material includes a first fullerene and/or a first fullerene derivative and/or a raw material for synthesizing the first fullerene derivative, and the second raw material includes at least one of a second fullerene and a second fullerene derivative used as a doping material.

In the preparation method, the doping material is added to the reaction system before the heat treatment, so that the doping material can be more uniformly dispersed in the reaction system. In the formed fullerene composite material, the doping material is more uniformly doped into the first fullerene derivative, which can reduce a risk of agglomeration of the doping material and is beneficial to improving doping uniformity.

In some implementations, the raw material for synthesizing the first fullerene derivative includes a third fullerene, 5-phenyl-5-(p-toluenesulfonylhydrazide) methyl pentanoate, and a base; and the mixing a first raw material and a second raw material in a first solvent includes:
dispersing the 5-phenyl-5-(p-toluenesulfonylhydrazide) methyl pentanoate and the base in a first sub-solvent, and dispersing the second raw material and the third fullerene in a second sub-solvent, and mixing the two solution systems.

In some implementations, a molar ratio of the third fullerene to the doping material to the 5-phenyl-5-(p-toluenesulfonylhydrazide) methyl pentanoate to the base is 1:(10-5 to 10-2):(1 to 2):(1 to 10).

In some implementations, the first sub-solvent includes pyridine.

In some implementations, the second sub-solvent includes at least one of toluene, xylene, chlorobenzene, and dichlorobenzene.

In some implementations, the base includes at least one of sodium methoxide, cesium carbonate, sodium hydroxide, and potassium hydroxide.

In some implementations, the first solvent includes at least one of toluene, xylene, chlorobenzene, and dichlorobenzene.

In some implementations, a temperature of the heat treatment ranges from 100°C to 300°C.

In some implementations, a time of the heat treatment ranges from 24 h to 60 h.

In some implementations, the heat treatment includes heating under reflux.

This application further provides a perovskite solar cell, including a first electrode, a perovskite layer, an electron transport layer, and a second electrode stacked in sequence, where
the electron transport layer includes the fullerene composite material; or
the electron transport layer includes a fullerene composite material obtained through the preparation method.

In the foregoing perovskite solar cell, the electron transport layer includes the fullerene composite material, which can improve performance of the perovskite solar cell.

In some implementations, a thickness of the electron transport layer ranges from 10 nm to 100 nm.

This application further provides a preparation method for a perovskite solar cell, including the following steps:
preparing a perovskite layer on a first electrode;
preparing an electron transport layer on the perovskite layer; and
preparing a second electrode on the electron transport layer, where
the electron transport layer includes a fullerene composite material, where the fullerene composite material includes a host material and a doping material, where the host material includes at least one of a first fullerene and a first fullerene derivative, the doping material is selected from at least one of a second fullerene and a second fullerene derivative, and a molecular structure of the doping material is partially embedded in a lattice of the first fullerene and/or the first fullerene derivative.

This application further provides an electrical device, including the perovskite solar cell.

### BRIEF DESCRIPTION OF THE DRAWINGS

To describe the technical solutions of this application more clearly, the following outlines the accompanying drawings to be used in this application. Evidently, the accompanying drawings outlined below are merely some implementations of this application. A person of ordinary skill in the art may further derive other accompanying drawings from such accompanying drawings without making any creative efforts.

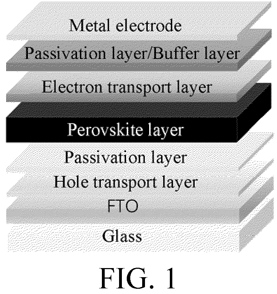

FIG. 1 is a schematic structural diagram of an exemplary perovskite solar cell.

To better describe and illustrate embodiments and/or examples of the present invention disclosed herein, reference may be made to one or more accompanying drawings. Additional details or examples used to describe the accompanying drawings are not to be considered as limiting the scope of any of the disclosure, currently described embodiments and/or examples, and the best modes of the present invention currently understood.

### DETAILED DESCRIPTION

A perovskite solar cell and a preparation method therefor and an electrical device according to this application are further described below in detail with reference to specific embodiments. The present invention may be implemented in many different forms, and is not limited to the implementations described in this specification. On the contrary, the objective of providing these implementations is to achieve a clear and complete comprehension of the disclosed content of the present invention.

Details of one or more embodiments of this application are provided in the accompanying drawings and descriptions below. Other features, objectives, and advantages of this application will become apparent from the specification, the accompanying drawings, and the claims.

Unless otherwise defined, all technical and scientific terms used in this specification have the same meanings as those generally understood by a person skilled in the art to which the present invention belongs. In this specification, the terms used in the specification of the present invention are only used for the purpose of describing the specific embodiments, rather than limiting the present invention.

A "range" disclosed in this application is defined in a form of a lower limit and an upper limit. A given range is defined by selecting a lower limit and an upper limit, and the selected lower limit and upper limit define boundaries of a particular range. A range defined in this manner may be inclusive or exclusive of end values, and may be arbitrarily combined, that is, any lower limit may be combined with any upper limit to form a range. For example, if a range of 60 to 120 and a range of 80 to 110 are listed for a particular parameter, it is understood that a range from 60 to 110 and a range from 80 to 120 are also contemplated. In addition, if minimum range values of 1 and 2 are listed and maximum range values of 3, 4, and 5 are listed, the following ranges are all contemplated: 1 to 3, 1 to 4, 1 to 5, 2 to 3, 2 to 4, and 2 to 5. In this application, unless otherwise specified, a numerical range "a to b" represents an abbreviated representation of any combination of real numbers between a to b, where both a and b are real numbers. For example, a numerical range "0 to 5" represents that all real numbers between "0 to 5" have been listed in this specification, and "0 to 5" is just an abbreviated representation of a combination of these numerical values. In addition, when it is stated that a parameter is an integer greater than or equal to 2, it is equivalent to disclosing that the parameter is, for example, an integer such as 2, 3, 4, 5, 6, 7, 8, 9, 10, 11, and 12.

Unless otherwise specified, all embodiments and optional embodiments of this application may be combined with each other to form new technical solutions.

Unless otherwise specified, all technical features and optional technical features of this application may be combined with each other to form new technical solutions.

Unless otherwise specified, all steps of this application may be performed sequentially or randomly, and preferably sequentially. For example, the phrase "the method includes step (a) and step (b)" means that the method may include step (a) and step (b) performed sequentially, or may include step (b) and step (a) performed sequentially. For example, the phrase "the method may further include step (c)" means that step (c) may be added to the method in any order. For example, the method may include step (a), step (b), and step (c), or may include step (a), step (c), and step (b), or may include step (c), step (a), and step (b).

Unless otherwise specified, the terms such as "include", "comprise", and their variants mentioned in this application may be open-ended or closed-ended. For example, the terms such as "include", "comprise", and their variants may mean that other components not listed may be further included or comprised, or only the listed components may be included or comprised.

Unless otherwise specified, the term "or" is inclusive in this application. For example, a phrase "A or B" means "A, B, or both A and B". More specifically, the condition "A or B" is satisfied when any one of the following conditions is satisfied: A is true (or present) and B is false (or absent); A is false (or absent) and B is true (or present); or both A and B are true (or present).

Unless otherwise specified, in this application, the term "room temperature" generally refers to 4°C to 30°C, and preferably 25±5°C.

This application provides a fullerene composite material, including a host material and a doping material, where the host material includes at least one of a first fullerene and a first fullerene derivative, the doping material is selected from at least one of a second fullerene and a second fullerene derivative, and a molecular structure of the doping material is partially embedded in a lattice of the first fullerene and/or the first fullerene derivative.

In the fullerene composite material according to this application, the host material including the first fullerene and/or the first fullerene derivative is doped with at least one of the second fullerene and the second fullerene derivative, and in the composite material, the molecular structure of the doping material is partially embedded in the lattice of the first fullerene and/or the first fullerene derivative, which can improve conductivity of the fullerene composite material and improve photoelectric conversion efficiency of the perovskite solar cell.

In the fullerene derivative, due to presence of branched chains, distances between molecules may increase, which makes it difficult to extract carriers and restricts improvement of conductivity of the fullerene derivative. The molecular structure of the doping material is partially embedded in the lattice of the first fullerene and/or the first fullerene derivative, which can reduce the distances between molecules, improve an electron transport capacity, and improve conductivity of the fullerene composite material.

It can be understood that the first fullerene and the second fullerene may be the same or different. The first fullerene derivative and the second fullerene derivative may be the same or different.

In some implementations, the first fullerene includes at least one of C50, C60, and C70. The first fullerene derivative includes at least one of PC51BM, PC52BM, PC61BM, PC62BM, PC71BM, and PC72BM. The second fullerene includes at least one of C50, C60, and C70. The second fullerene derivative includes at least one of PC51BM, PC52BM, PC61BM, PC62BM, PC71BM, and PC72BM. The fullerenes and fullerene derivatives are widely available and are easy to obtain.

It can also be understood that fullerene derivatives are usually obtained by modifying fullerenes. The lattice of the fullerene is usually spherical, so that the lattice of the fullerene derivative also mainly exhibits a spherical shape. When the molecular structure of the doping material is partially embedded in the lattice of the first fullerene and/or the first fullerene derivative, it can be exhibited as that the molecular structure of the doping material is partially embedded in a spherical lattice of the first fullerene and/or the first fullerene derivative. Optionally, when the doping material has a side chain, the side chain of the doping material is embedded in the lattice of the first fullerene and/or the first fullerene derivative. It can be understood that fullerene derivatives are usually obtained by modifying fullerenes such as C50, C60, and C70.

It can also be understood that when the molecular structure of the doping material is partially embedded in the lattice of the first fullerene and/or the first fullerene derivative, an additional structure of the molecular structure of the doping material can extend from the lattice of the first fullerene and/or the first fullerene derivative or can be adhered to an outer surface of the lattice of the first fullerene and/or the first fullerene derivative.

In some implementations, a mass percentage of the doping material based on a total mass of the first fullerene and/or the first fullerene derivative and the doping material ranges from 0.0001% to 1%. Optionally, the mass percentage of the doping material based on the total mass of the first fullerene and/or the first fullerene derivative and the doping material is 0.0001%, 0.001%, 0.01%, 0.02%, 0.03%, 0.05%, 0.08%, 0.1%, 0.2%, 0.3%, 0.4%, 0.5%, 0.6%, 0.7%, 0.8%, 0.9%, 1%, or the like. If the mass percentage of the doping material is too small, a doping effect is poor, and if the mass percentage of the doping material is too large, an intrinsic structure of the host material may be excessively affected.

Optionally, a type and a content of the doping material can be measured through liquid chromatography. Optionally, conditions of the liquid chromatography are as follows: A chromatography column is a C18 directional column with a length of 15 cm, a mobile phase is toluene:methanol=1:2, and a detector is a DAD detector.

In this application, the mass percentage of the doping material based on the total mass of the first fullerene and/or the first fullerene derivative and the doping material may be denoted as a doping amount of the doping material.

In some implementations, a fullerene composite material includes a host material and a doping material, where the host material is selected from at least one of a first fullerene and a first fullerene derivative, the doping material is selected from at least one of a fullerene and a second fullerene derivative, and a molecular structure of the doping material is partially embedded in a lattice of the first fullerene and/or the first fullerene derivative. Optionally, a mass percentage of the doping material based on a total mass of the first fullerene and/or the first fullerene derivative and the doping material ranges from 0.0001% to 1%. Optionally, the mass percentage of the doping material based on the total mass of the first fullerene and/or the first fullerene derivative and the doping material is 0.0001%, 0.001%, 0.01%, 0.02%, 0.03%, 0.05%, 0.08%, 0.1%, 0.2%, 0.3%, 0.4%, 0.5%, 0.6%, 0.7%, 0.8%, 0.9%, 1%, or the like.

This application further provides a preparation method for a fullerene composite material. The preparation method for a fullerene composite material includes the following step: mixing a first raw material and a second raw material in a first solvent, and performing heat treatment on an obtained mixed system, where the first raw material includes a first fullerene and/or a first fullerene derivative and/or a raw material for synthesizing the first fullerene derivative, and the second raw material includes at least one of a fullerene and a second fullerene derivative used as a doping material.

When the fullerene composite material is prepared, agglomeration of the doping material is an important factor affecting performance of the composite material. In the preparation method, the doping material is added to the reaction system before the heat treatment, so that the doping material can be more uniformly dispersed in the reaction system. In the formed fullerene composite material, the doping material is more uniformly doped into the first fullerene derivative, which can reduce a risk of agglomeration of the doping material and is beneficial to improving doping uniformity.

In some implementations, the first raw material includes a raw material for synthesizing the first fullerene derivative, where the raw material for synthesizing the first fullerene derivative includes a third fullerene, 5-phenyl-5-(p-toluenesulfonylhydrazide) methyl pentanoate, and a base; and the mixing a first raw material and a second raw material in a solvent includes: dispersing the 5-phenyl-5-(p-toluenesulfonylhydrazide) methyl pentanoate and the base in a first sub-solvent, and dispersing the second raw material and the third fullerene in a second sub-solvent, and mixing the two solution systems.

Optionally, the third fullerene includes at least one of C50, C60, and C70. Optionally, the first fullerene, the second fullerene, and the third fullerene may not be completely the same or may be completely the same.

In some implementations, a molar ratio of the third fullerene to the doping material to the 5-phenyl-5-(p-toluenesulfonylhydrazide) methyl pentanoate to the base is 1:(10⁻⁵ to 10⁻²):(1 to 2):(1 to 10). Optionally, the molar ratio of the third fullerene to the doping material to the 5-phenyl-5-(p-toluenesulfonylhydrazide) methyl pentanoate to the base is 1:10⁻⁵:1:1, 1:10⁻⁵:1:5, 1:10⁻⁵:1:8, 1:10⁻⁴:1:1, 1:10⁻⁴:1:5, 1:10⁻⁴:1:8, 1:10⁻³:1:1, 1:10⁻³:1:5, 1:10⁻³:1:8, 1:10⁻²:1:1, 1:10⁻²:1:5, 1:10⁻²:1:8, or the like.

In some implementations, the first sub-solvent includes pyridine.

Optionally, the second sub-solvent includes at least one of toluene, xylene, chlorobenzene, and dichlorobenzene. The xylene includes at least one of o-xylene, m-xylene, and p-xylene. The dichlorobenzene includes at least one of o-dichlorobenzene, m-dichlorobenzene, and p-dichlorobenzene.

Optionally, the base includes at least one of sodium methoxide, cesium carbonate, sodium hydroxide, and potassium hydroxide.

Optionally, the raw material for synthesizing the first fullerene derivative includes a third fullerene, 5-phenyl-5-(p-toluenesulfonylhydrazide) methyl pentanoate, and a base; and the mixing a first raw material and a second raw material in a solvent includes: adding the 5-phenyl-5-(p-toluenesulfonylhydrazide) methyl pentanoate into a first sub-solvent, then, adding the base, and stirring, vacuumizing, and releasing a gas to place the solution system in an argon atmosphere; and adding the second raw material and the third fullerene into a second sub-solvent, and then, adding the obtained solution into the solution system including the first sub-solvent.

Optionally, the raw material for synthesizing the first fullerene derivative includes a third fullerene, 5-phenyl-5-(p-toluenesulfonylhydrazide) methyl pentanoate, and a base. After the mixing a first raw material and a second raw material in a first solvent, and performing heat treatment on an obtained mixed system, the preparation method further includes: removing the solvent from a product obtained after the heat treatment, and then, separating the product. Optionally, the solvent may be removed through rotary evaporation. Optionally, the product may be separated through column chromatography.

In some implementations, the first raw material is a first fullerene derivative. A mass percentage of the doping material based on a total mass of the first fullerene derivative and the doping material ranges from 0.0001% to 1%. Optionally, the mass percentage of the doping material based on the total mass of the first fullerene derivative and the doping material is 0.0001%, 0.001%, 0.01%, 0.02%, 0.03%, 0.05%, 0.08%, 0.1%, 0.2%, 0.3%, 0.4%, 0.5%, 0.6%, 0.7%, 0.8%, 0.9%, 1%, or the like.

Optionally, the first raw material is a first fullerene derivative. After the mixing a first raw material and a second raw material in a first solvent, and performing heat treatment on an obtained mixed system, the preparation method further includes: concentrating a product obtained after the heat treatment, and then, adding methanol to precipitate. The fullerene composite material is obtained through precipitation.

In some implementations, the first solvent includes at least one of toluene, xylene, chlorobenzene, and dichlorobenzene. The xylene includes at least one of o-xylene, m-xylene, and p-xylene. The dichlorobenzene includes at least one of o-dichlorobenzene, m-dichlorobenzene, and p-dichlorobenzene.

In some implementations, a temperature of the heat treatment ranges from 100°C to 300°C. A time of the heat treatment ranges from 24 h to 60 h. The heat treatment includes heating under reflux. Optionally, the temperature of the heat treatment is 100°C, 120°C, 150°C, 180°C, 200°C, 220°C, 250°C, 280°C, 300°C, or the like. The time of the heat treatment is 24 h, 30 h, 36 h, 48 h, 60 h, or the like.

In some implementations, a preparation method for the 5-phenyl-5-(p-toluenesulfonylhydrazide) methyl pentanoate includes: mixing methyl 4-benzoylbutyrate, p-toluenesulfonyl hydrazide, and methanol and heating under reflux for reaction, cooling to room temperature, storing in a shade, then transferring to -15°C and storing in a shade overnight, performing suction filtration, recrystallizing the product in methanol, and vacuumizing the product at 40°C overnight to obtain 5-phenyl-5-(p-toluenesulfonylhydrazide) methyl pentanoate as a white solid. Optionally, a time of the heating under reflux for reaction is 12 h, and after being cooled to room temperature, the product is stored in a shade for 24 h.

In some implementations, a preparation method for the methyl 4-benzoylbutyrate includes: dissolving benzoylbutyric acid in chlorobenzene, adding methanol, stirring until they are completely dissolved, adding hydrochloric acid dropwise, continuing stirring, and heating under reflux for reaction overnight, stopping stirring, neutralizing with saturated sodium carbonate until there are no bubbles, separating oil and water, extracting aqueous phases with ethyl acetate, combining organic phases, removing water with anhydrous calcium chloride, distilling under reduced pressure to remove the solvent, and eluting and separating the product in a silica gel column, where an eluent is ethyl acetate and n-hexane, where a volume ratio of the ethyl acetate to the n-hexane is 3: 1, to obtain methyl 4-benzoylbutyrate as a light yellow oily liquid.

This application further provides an electron transport film. The electron transport film includes the foregoing fullerene composite material or a fullerene composite material obtained through the foregoing preparation method for a fullerene composite material. Optionally, a thickness of the electron transport film ranges from 10 nm to 100 nm. Further optionally, the thickness of the electron transport film is 10 nm, 20 nm, 30 nm, 40 nm, 50 nm, 60 nm, 70 nm, 80 nm, 90 nm, 100 nm, or the like.

This application further provides an electron transport slurry. The electron transport slurry includes a second solvent and further includes the foregoing fullerene composite material or a fullerene composite material obtained through the foregoing preparation method for a fullerene composite material. Optionally, the second solvent in the electron transport slurry includes at least one of toluene, xylene, chlorobenzene, and dichlorobenzene. The xylene includes at least one of o-xylene, m-xylene, and p-xylene. The dichlorobenzene includes at least one of o-dichlorobenzene, m-dichlorobenzene, and p-dichlorobenzene.

This application further provides a perovskite solar cell. The perovskite solar cell includes a first electrode layer, a perovskite layer, an electron transport layer, and a second electrode layer that are stacked in sequence. The electron transport layer includes the foregoing fullerene composite material, or the electron transport layer includes a fullerene composite material obtained through the foregoing preparation method, or the electron transport layer includes the foregoing electron transport film, or the electron transport layer is prepared from a material including the foregoing electron transport slurry. In the perovskite solar cell, the electron transport layer includes the fullerene composite material, which can improve performance of the perovskite solar cell.

Optionally, the electron transport layer is prepared by spin coating a material including the foregoing electron transport slurry and annealing. Further, the electron transport layer is prepared by spin coating the foregoing electron transport slurry and annealing.

Optionally, a thickness of the electron transport layer ranges from 10 nm to 100 nm. Optionally, the thickness of the electron transport layer is 10 nm, 20 nm, 30 nm, 40 nm, 50 nm, 60 nm, 70 nm, 80 nm, 90 nm, 100 nm, or the like.

Optionally, the electron transport layer is obtained by spin coating a material including the foregoing electron transport slurry and annealing.

Optionally, the first electrode is a transparent electrode or a metal electrode. The second electrode is a transparent electrode or a metal electrode. Further optionally, the first electrode is different from the second electrode. Optionally, the transparent electrode is a transparent glass electrode.

In some implementations, the first electrode is generally made of transparent conductive glass. Optionally, the first electrode is selected from at least one of fluorine-doped tin oxide (FTO), indium tin oxide (ITO), aluminum-doped zinc oxide (AZO), boron-doped zinc oxide (BZO), indium zinc oxide (IZO), and indium tungsten oxide (IWO). Optionally, a thickness of the first electrode layer ranges from 100 nm to 1000 nm, and optionally, from 300 nm to 800 nm.

In some implementations, the second electrode is generally a metal electrode. Optionally, the second electrode is selected from at least one of Au, Ag, Cu, Al, Ni, Cr, Bi, Pt, Mg, Mo, W, and alloys thereof. Optionally, a thickness of the second electrode ranges from 20 nm to 200 nm, optionally, from 60 nm to 100 nm, and further optionally, from 70 nm to 90 nm. It may be understood that the perovskite layer is a light-absorbing layer and includes a perovskite material. Optionally, a chemical formula of a material of the perovskite layer is ABX₃ or A₂CDX₆, where:

A is an inorganic or organic or organic-inorganic hybrid cation, including at least one of an organic amine cation, a Cs cation, a K cation, a Rb cation, and a Li cation. The organic amine cation is selected from (NR1R2R3R4)⁺, (R1R2N=CR3R4)⁺, (R1R2N-C(R5)=NR3R4)⁺, or (R1R2N-C (NR5R6)=R3R4)⁺, where R1, R2, R3, R4, R5, and R6 are each independently selected from H, a substituted or unsubstituted C1-20 alkyl group, or a substituted or unsubstituted aryl group. A is optionally at least one of a methylamino group (CH₃NH₃⁺)(MA⁺), a formamidine group (HC(NH₂)₂⁺)(FA⁺), a cesium ion (Cs⁺), and rubidium (Rb⁺), and further optionally a methylamino group (CH₃NH₃⁺) or a methylamino group (HC(NH)₂)₂⁺).

B is an inorganic or organic or organic-inorganic hybrid cation, including at least one of lead, tin, zinc, titanium, antimony, bismuth, nickel, iron, cobalt, silver, copper, gallium, germanium, magnesium, calcium, indium, aluminum, manganese, chromium, molybdenum, and europium, and optionally, at least one of divalent metal ions Pb²⁺ and Sn²⁺.

C is an inorganic or organic or organic-inorganic hybrid cation, and optionally, a monovalent metal ion such as Ag⁺.

D is an inorganic or organic or organic-inorganic hybrid cation, and optionally a trivalent metal ion such as a bismuth cation Bi³⁺, an antimony cation Sb³⁺, or an indium cation In³⁺.

X is an inorganic or organic or organic-inorganic hybrid anion, optionally, one or more of a halogen anion and a carboxylate anion, and further optionally, a bromide ion (Br⁻) or an iodide ion (I⁻).

In some implementations, a band gap of the perovskite layer ranges from 1.20 eV to 2.30 eV.

In some implementations, a thickness of the perovskite layer ranges from 200 nm to 800 nm, and optionally, from 400 nm to 600 nm.

In some implementations, the perovskite solar cell further includes a hole transport layer, where the hole transport layer is located between the first electrode and the perovskite layer. Optionally, the hole transport layer may be made of at least one of the following materials, derivatives thereof, and materials obtained by doping or passivating the following materials: poly[bis(4-phenyl)(2,4,6-trimethylphenyl)amine] (PTAA), poly(3-hexylthiophene) (P3HT), triptycene-cored triphenylamine (H101), 3,4-ethylenedioxythiophene-methoxytriphenylamine (EDOT-OMeTPA), N-(4-aniline)carbazole-spirobifluorene (CzPAF-SBF), poly(3,4-ethylenedioxythiophene):poly(styrenesulfonate) (PEDOT:PSS), polythiophene, nickel oxide (NiOₓ), molybdenum oxide (MoO₃), cuprous iodide (CuI), cuprous oxide (CuO), and the like.

In some implementations, the perovskite solar cell further includes a first passivation layer, where the first passivation layer is located between the perovskite layer and the hole transport layer. Optionally, a material of the first passivation layer is PTAA.

In some implementations, the perovskite solar cell further includes a second passivation layer and/or a buffer layer, and the second passivation layer and/or the buffer layer is located between the electron transport layer and the second electrode. Optionally, materials of the second passivation layer and the buffer layer are bathocuproine (BCP).

It may be understood that the perovskite solar cell includes a conventional perovskite solar cell and an inverted perovskite solar cell. For the conventional perovskite solar cell, the conventional perovskite solar cell includes a transparent electrode as well as an electron transport layer, a perovskite layer, a hole transport layer, and a metal electrode sequentially stacked on the transparent electrode. For the inverted perovskite solar cell, the inverted perovskite solar cell includes a transparent electrode as well as a hole transport layer, a perovskite layer, an electron transport layer, and a metal electrode sequentially stacked on the transparent electrode. In this application, by selecting the materials of the first electrode and the second electrode, the corresponding conventional perovskite solar cell and inverted perovskite solar cell can be obtained.

Referring to FIG. 1, a structure of a perovskite solar cell according to an embodiment of this application is shown. A first electrode is made of FTO, and a hole transport layer, a passivation layer, a perovskite layer, an electron transport layer, a passivation layer or buffer layer, and a metal electrode are stacked in sequence on the first electrode. A glass substrate is arranged on a surface of the FTO away from the perovskite layer.

This application further provides a preparation method for a perovskite solar cell. The preparation method for a perovskite solar cell includes the following steps: preparing a perovskite layer on a first electrode; preparing an electron transport layer on the perovskite layer; and preparing a second electrode on the electron transport layer, where the electron transport layer includes a fullerene composite material, where the fullerene composite material includes a host material and a doping material, where the host material includes a first fullerene derivative, the doping material is selected from at least one of a fullerene and a second fullerene derivative, and a molecular structure of the doping material is partially embedded in a lattice of the first fullerene derivative.

Optionally, the perovskite layer is prepared by spin coating and annealing. Optionally, the electron transport layer is prepared by spin coating and annealing. Optionally, the second electrode is prepared through evaporation.

Optionally, the electron transport layer is prepared by spin coating the foregoing electron transport slurry and annealing.

This application further provides an electrical device. The electrical device includes the foregoing perovskite solar cell. Optionally, the electrical device is an electrical device in, for example, the field of communication, the field of transportation, the field of industry and agriculture, or the field of lighting. The electrical device may include, for example, a satellite, a communication device, traffic lights, a lighthouse, a wireless phone booth, a monitoring device in the field of oil drilling, a power supply system, a camping light, an electric vehicle, and an electronic device charger.

### Examples

The examples of this application are described below. The examples described below are exemplary and are only intended to explain this application, and cannot be understood as limitations to this application. Where specific technologies or conditions are not indicated in the examples, they are carried out according to technologies or conditions described in the literature in the art or according to the product specifications. The reagents or instruments for which no manufacturers are noted are all common products commercially available from the market.

### Example 1

Steps for preparing a fullerene composite material in this example were as follows:

### S101: Synthesis of methyl 4-benzoylbutyrate:

A clean and dry 500 mL, two-necked flask was taken, 19.25 g (0.1 mol) of benzoylbutyric acid was added and dissolved in 300 mL of chlorobenzene, 100 mL of methanol was added, the mixture was stirred until they were completely dissolved, 30 mL of hydrochloric acid was added dropwise, the stirring was continued, heating under reflux was performed for reaction overnight, it was determined, through thin-layer chromatography, that the reaction was complete, then, the stirring was stopped, the product was neutralized with saturated sodium carbonate until there were no bubbles, oil and water were separated, aqueous phases were extracted with ethyl acetate, organic phases were combined, water was removed with anhydrous calcium chloride, the product was distilled under reduced pressure to remove the solvent, and the product was eluted and separated in a silica gel column, where an eluent was ethyl acetate and n-hexane (at a volume ratio of 3:1), to obtain 18 g of methyl 4-benzoylbutyrate as a light yellow oily liquid.

### S102: Synthesis of methyl 5-phenyl-5-(p-toluenesulfonylhydrazide) methyl pentanoate:

10.5 g (0.05 mol) of methyl 4-benzoylbutyrate, 11. 5 g (0.06 mol) of p-toluenesulfonyl hydrazide, and 200 ml of methanol were heated under reflux in a single-necked round-bottom flask, reacted for 12 h, cooled to room temperature, stored in a shade for 24 h, and then transferred to -15°C and stored in a shade overnight, suction filtration was performed, and the product was recrystallized in methanol, and vacuumized at 40°C overnight to obtain 18.2 g of 5-phenyl-5-(p-toluenesulfonylhydrazide) methyl pentanoate as a white solid.

### S103: Synthesis of F1-OMe:

A clean and dry 1 L, three-necked flask was taken, 11.25 g (0.03 mol) of 5-phenyl-5-(p-toluenesulfonylhydrazide) methyl pentanoate was dissolved in 250 mL of pyridine, 1.75 g of sodium methoxide was added, the mixed was stirred, vacuumization was performed, and a gas was released, to store the product under an argon atmosphere; another clean and dry round-bottomed flask was taken, 10.8 g of C60 was added and dissolved in 300 mL of o-dichlorobenzene, the solution was added into the foregoing three-necked flask dropwise, heating under reflux was performed for 24 h, the product was rotary evaporated to remove most of the solvent, and the product was separated through column chromatography to obtain 6.83 g of a product F1-OMe.

### S104: Fullerene composite material:

6.83 g of F1-OMe and a doping material C50 were dissolved in o-dichlorobenzene, the solution was heated under reflux for reaction for 48 h, and after the product was concentrated, methanol was added to precipitate, to obtain a fullerene composite material. In this example, the doping material was C50, the host material was PC61BM, and in the obtained fullerene composite material, the mass percentage of the doping material C50 in the fullerene composite material was 0.1%, that is, the doping amount was 0.1%.

In this embodiment, steps for preparing a perovskite solar cell were as follows:
S201: 20 pieces of FTO conductive glass with a specification of 2.0 cm*2.0 cm were taken, and 0.35 cm of FTO was removed from each of two ends by laser etching, to expose a glass substrate; the etched FTO conductive glass was ultrasonically cleaned with water, acetone, and isopropanol in sequence; the solvent on the cleaned FTO conductive glass was blow-dried by a nitrogen gun, and the cleaned FTO conductive glass was put into an ultraviolet ozone machine for ultraviolet ozone cleaning treatment.
S202: An FTO substrate obtained after the ultraviolet ozone cleaning treatment was spin-coated with 10 mg/mL nickel oxide nanoparticles (with water as a solvent) at a rate of 4000 rpm and annealed on a hot plate at 100°C for 30 minutes to form a hole transport layer.
S203: PTAA was dissolved in chlorobenzene at a concentration of 1 mg/mL, after being stirred, the solution was filtered with a 0.45 µm filter membrane, 50 µL of the filtered solution was added dropwise onto the hole transport layer and spin-coated at a rate of 4000 rpm, and the product was annealed on a hot plate at 100°C for 10 minutes to obtain a passivation layer.
S204: 223 mg of lead iodide PbI2, 80 mg of formamidine iodide (FAI), 15 mg of chloromethylamine (MACl) were weighed and dissolved in a mixed solution of 0.8 mL of DMF and 0.2 mL of DMSO, the solution was stirred for 3 h and filtered with a 0.22 µm organic filter membrane to obtain a perovskite precursor solution, the perovskite precursor solution was spin-coated on the passivation layer at 3000 rpm, the product was annealed at 120°C for 30 min, and cooled to room temperature to form a perovskite layer, where an active material in the perovskite layer was a FA system with a thickness of 500 nm.
S205: The fullerene composite material obtained in S104 was dissolved in chlorobenzene to form an electron transport slurry with a concentration of 20 mg/mL, the electron transport slurry was spin-coated on the perovskite layer at 1500 rpm, and the product was annealed at 100°C for 10 min to form an electron transport layer with a thickness of 50 nm. Next, a passivation material BCP solution thereof was spin-coated at 5000 rpm, where the BCP solution was prepared by dissolving BCP in isopropanol with a concentration of 0.5 mg/mL to form a passivation layer with a thickness of 5 nm. Then, the product was put into an evaporation coating machine, and a metal electrode Ag was evaporated to obtain a perovskite solar cell.

### Example 2

This example differed from Example 1 in that the doping material was PC51BM. PC51BM was prepared through S101 to S103 in Example 1 and by replacing C60 in S103 with C50.

During preparation of the fullerene composite material, the doping material C50 in S104 in Example 1 was replaced with PC51BM.

In this example, the doping material was PC51BM, the host material was PC61BM, and in the obtained fullerene composite material, the mass percentage of the doping material PC51BM in the fullerene composite material was 0.05%, that is, the doping amount was 0.05%.

### Example 3

This example differed from Example 1 in that C60 in S103 was replaced with C50 and C60, where a total mass of C50 and C60 was equal to a mass of C60 in S103 in Example 1, and based on the total mass of C50 and C60, a mass percentage of C50 was 0.1%.

In this example, the doping material was PC51BM, the host material was PC61BM, and in the obtained fullerene composite material, the mass percentage of the doping material PC51BM in the fullerene composite material was 0.05%, that is, the doping amount was 0.05%.

### Example 4

This example differed from Example 1 in that C60 in S103 was replaced with C50, C60, and C70, where a total mass of 50, C60, and C70 was equal to a mass of C60 in S103 in Example 1, and based on the total mass of C50, C60, and C70, a mass percentage of C50 was 0.1%, and a mass percentage of C70 was 0.03%.

In this example, the doping material was PC51BM and C71BM, the host material was PC61BM, and in the obtained fullerene composite material, the mass percentage of the doping material PC51BM in the fullerene composite material was 0.05%, and the mass percentage of the doping material PC71BM in the fullerene composite material was 0.015%. That is, the doping amount of PC51BM was 0.05%, and the doping amount of PC71BM was 0.03%.

### Example 5

This example differed from Example 1 in that an addition amount of the doping material C50 in Step S104 was adjusted. The doping material was C50, the host material was PC61BM, and in the obtained fullerene composite material, the mass percentage of the doping material C50 in the fullerene composite material was 0.0001%.

### Example 6

This example differed from Example 1 in that an addition amount of the doping material C50 in Step S104 was adjusted. In this example, the doping material was C50, the host material was PC61BM, and in the obtained fullerene composite material, the mass percentage of the doping material C50 in the fullerene composite material was 1%.

### Example 7

This example differed from Example 1 in that C50 was replaced with C70.

### Example 8

This example differed from Example 1 in that an addition amount of the doping material C50 in Step S104 was adjusted. In this example, the doping material was C50, the host material was PC61BM, and in the obtained fullerene composite material, the mass percentage of the doping material C50 in the fullerene composite material was 0.00005%.

### Example 9

This example differed from Example 1 in that an addition amount of the doping material C50 in Step S104 was adjusted. In this example, the doping material was C50, the host material was PC61BM, and in the obtained fullerene composite material, the mass percentage of the doping material C50 in the fullerene composite material was 1.3%.

### Example 10

This example differed from Example 1 in that S101 to S103 are not performed, and in S104, C60 and a doping material C50 were dissolved in o-dichlorobenzene, the solution was heated under reflux for reaction for 48 h, and after the product was concentrated, methanol was added to precipitate, to obtain a fullerene composite material. The doping material was C50, the host material was C60, and in the obtained fullerene composite material, the mass percentage of the doping material C50 in the fullerene composite material was 0.1%.

### Comparative example 1

This comparative example differed from Example 1 in that in S104, no C50 was added, that is, PC61BM was not doped.

### Comparative example 2

This comparative example differed from Example 1 in that PC61BM was directly mixed with C50 to obtain a fullerene composite material. A mass percentage of C50 in the fullerene composite material was 0.1%.

### Comparative example 3

Compared with Example 1, no fullerene composite material was prepared, and in a perovskite solar cell, an electron transport layer was prepared by evaporating C60.

### Test Examples:

Performance of batteries was tested under radiation of standard simulated sunlight (AM 1.5 G, 100 mW/cm²), to obtain I-V curves. Short-circuit currents Jsc (in mA/cm²), open-circuit voltages Voc (in V), maximum light output currents Jmpp (in mA), and maximum light output voltages Vmpp (in V) can be obtained based on the I-V curves and data fed back by a test device. Fill factors FF of the batteries were calculated using the formula FF=Jsc×Voc/(Jmpp×Vmpp), in %. Photoelectric conversion efficiency PCE of the batteries was calculated using the formula PCE=Jsc×Voc×FF/Pw, in %, where Pw represented an input power, in mW.

Doping amounts of doping materials and test results of the batteries in the examples and comparative examples are shown in Table 1.

**Table 1**

| | Host material | Doping material and doping amount | Short-circuit current | Open-circuit voltage | Photoelectric conversion efficiency |
|---|---|---|---|---|---|
| Example 1 | PC61BM | C50 0.1% | 21.12 | 1.07 | 16.85% |
| Example 2 | PC61BM | PC51BM 0.05% | 21.33 | 1.08 | 17.01% |
| Example 3 | PC61BM | PC51BM 0.05% | 21.38 | 1.09 | 17.21% |
| Example 4 | PC61BM | PC51BM 0.05% | 21.54 | 1.1 | 17.89% |
| | | PC71BM 0.03% | | | |
| Example 5 | PC61BM | C50 0.0001% | 21.01 | 1.05 | 16.18% |
| Example 6 | PC61BM | C50 1% | 21.21 | 1.06 | 16.99% |
| Example 7 | PC61BM | C70 0.1% | 21.21 | 1.12 | 18.01% |
| Example 8 | PC61BM | C50 0.00005% | 20.62 | 1.03 | 15.88% |
| Example 9 | PC61BM | C50 1.3% | 21.18 | 1.06 | 16.83% |
| Example 10 | C60 | C50 0.1% | 21.22 | 1.07 | 17.01% |
| Comparative example 1 | PC61BM | / | 20.23 | 1.05 | 15.23% |
| Comparative example 2 | / | / | 20.58 | 1.03 | 15.42% |
| Comparative example 3 | / | / | 20.49 | 1.04 | 15.62% |

It can be seen from Table 1 that the perovskite solar cells in the examples achieve higher photoelectric conversion efficiency than the comparative examples. For example, it can be seen from the examples, Comparative example 1, and Comparative example 3 that a perovskite solar cell in which an electron transport layer includes a doping material has higher photoelectric conversion efficiency. It can be seen from Example 1 and Comparative example 2 that, compared with the manner of directly mixing PC61BM and C50, using the fullerene composite material obtained through the preparation method in Example 1 in the electron transport layer can obtain a perovskite solar cell with higher photoelectric conversion efficiency. Meanwhile, when a mass percentage of the doping material in the fullerene composite material ranges from 0.0001% to 1%, the perovskite solar cell can obtain higher photoelectric conversion efficiency.

It should be noted that the application is not limited to the foregoing implementations. The foregoing implementations are exemplary only, and any implementation within the scope of the technical solution of this application that has substantially the same composition and has the same effects as the technical idea is encompassed in the technical scope of this application. In addition, without departing from the gist of this application, various modifications that may be conceived by a person skilled in the art to the implementations, and other modes constructed by combining some of the constituent elements of the implementations are also included in the scope of this application.

## Claims

1. A fullerene composite material, comprising a host material and a doping material, wherein the host material comprises at least one of a first fullerene and a first fullerene derivative, the doping material is selected from at least one of a second fullerene and a second fullerene derivative, and a molecular structure of the doping material is partially embedded in a lattice of the first fullerene and/or the first fullerene derivative.

2. The fullerene composite material according to claim 1, wherein a mass percentage of the doping material based on a total mass of the first fullerene and/or the first fullerene derivative and the doping material ranges from 0.0001% to 1%.

3. The fullerene composite material according to claim 1 or 2, wherein the first fullerene comprises at least one of C50, C60 and C70, and the first fullerene derivative comprises at least one of PC51BM, PC52BM, PC61BM, PC62BM, PC71BM, and PC72BM.

4. The fullerene composite material according to any one of claims 1 to 3, wherein the second fullerene comprises at least one of C50, C60 and C70, and the second fullerene derivative comprises at least one of PC51BM, PC52BM, PC61BM, PC62BM, PC71BM, and PC72BM.

5. A preparation method for a fullerene composite material, comprising the following step:
mixing a first raw material and a second raw material in a first solvent, and performing heat treatment on an obtained mixed system, wherein
the first raw material comprises a first fullerene and/or a first fullerene derivative and/or a raw material for synthesizing the first fullerene derivative, and the second raw material comprises at least one of a second fullerene and a second fullerene derivative used as a doping material.

6. The preparation method for a fullerene composite material according to claim 5, wherein the raw material for synthesizing the first fullerene derivative comprises a third fullerene, 5-phenyl-5-(p-toluenesulfonylhydrazide) methyl pentanoate, and a base; and the mixing a first raw material and a second raw material in a first solvent comprises:
dispersing the 5-phenyl-5-(p-toluenesulfonylhydrazide) methyl pentanoate and the base in a first sub-solvent, and dispersing the second raw material and the third fullerene in a second sub-solvent, and mixing the two solution systems.

7. The preparation method for a fullerene composite material according to claim 6, wherein a molar ratio of the third fullerene to the doping material to the 5-phenyl-5-(p-toluenesulfonylhydrazide) methyl pentanoate to the base is 1:(10⁻⁵ to 10⁻²):(1 to 2):(1 to 10).

8. The preparation method for a fullerene composite material according to claim 6 or 7, wherein the first sub-solvent comprises pyridine; and/or
the second sub-solvent comprises at least one of toluene, xylene, chlorobenzene, and dichlorobenzene; and/or
the base comprises at least one of sodium methoxide, cesium carbonate, sodium hydroxide, and potassium hydroxide.

9. The preparation method for a fullerene composite material according to any one of claims 5 to 8, wherein the first solvent comprises at least one of toluene, xylene, chlorobenzene, and dichlorobenzene.

10. The preparation method for a fullerene composite material according to any one of claims 5 to 8, wherein the heat treatment satisfies at least one of the following characteristics:
(1) a temperature of the heat treatment ranges from 100°C to 300°C;
(2) a time of the heat treatment ranges from 24 h to 60 h; and
(3) the heat treatment comprises heating under reflux.

11. A perovskite solar cell, comprising a first electrode, a perovskite layer, an electron transport layer, and a second electrode stacked in sequence, wherein
the electron transport layer comprises the fullerene composite material according to any one of claims 1 to 4; or
the electron transport layer comprises a fullerene composite material obtained through the preparation method according to any one of claims 5 to 10.

12. The perovskite solar cell according to claim 11, wherein a thickness of the electron transport layer ranges from 10 nm to 100 nm.

13. A preparation method for a perovskite solar cell, comprising the following steps:
preparing a perovskite layer on a first electrode;
preparing an electron transport layer on the perovskite layer; and
preparing a second electrode on the electron transport layer, wherein
the electron transport layer comprises a fullerene composite material, wherein the fullerene composite material comprises a host material and a doping material, wherein the host material comprises at least one of a first fullerene and a first fullerene derivative, the doping material is selected from at least one of a second fullerene and a second fullerene derivative, and a molecular structure of the doping material is partially embedded in a lattice of the first fullerene and/or the first fullerene derivative.

14. An electrical device, comprising the perovskite solar cell according to claim 11 or 12.
